# EUROPEAN PATENT SPECIFICATION

(11) **EP 0 353 743 B1**
(45) Date of publication and mention of the grant of the patent: **28.04.1993**
(21) Application number: 89114294.5
(22) Date of filing: 02.08.1989
(51) Int. Cl.: C07C 271/68, C07C 239/00

(54) **Fluorinated alkoximines, their n-chloro. and n-bromo-derivatives, and process for the preparation thereof**
Fluorinierte Alkoximine, deren N-Chlor-und N-Brom Derivate sowie Verfahren zur Herstellung davon
Alkoximines fluorées, leurs dérivés n-chloro et n-bromo et procédé pour les préparer

(30) Priority: 03.08.1988 IT 2162788
(43) Date of publication of application: 07.02.1990
(73) Proprietor: AUSIMONT S.p.A., I-20121 Milano (IT)
(72) Inventor: Desmarteau, Darryl D., Dr., Clemson South Carolina 29631 (US); Kotun, Stefan P., Dr., Clemson South Carolina 29631 (US)
(74) Representative: Barz, Peter, Dr.

(56) References cited:
- CHEMICAL ABSTRACTS, vol. 96., no. 3, 18th January 1982, page 404, abstract no. 19610c, Columbus, Ohio, US; W.Y. LAM: "Some novel oxime ethers. The synthesis of 1,1-difluoro-2-alkoxymethanimines", & J. FLUORINE CHEM. 1981, 18(4-6), 441-5

## Description

The present invention relates to fluorinated alkoximines and to a process for their preparation. It also relates to N-fluoro-, N-chloro- and N-bromo-derivatives of the above alkoximines and to the preparation thereof.

One object of the present invention is to provide new fluorinated alkoximines.
Another object is to provide new N-chloro- and N-bromo-derivatives of said fluorinated alkoximines.
A further object is to provide a process for the preparation of said fluorinated alkoximines.
Still another object is to provide a process for preparing the N-chloro-, N-bromo- and N-fluoro-derivatives of said fluorinated alkoximines.

According to the first object of the present invention, there are provided fluorinated alkoximines of general formula:

Rₓ-CF=N-O-CZ₁Z₂-CF₃ (I)

wherein:
- Rₓ: is F or a perhalogenated alkyl group containing from 1 to 3 carbon atoms (e.g. CF₃ and C₂F₅), and
- Z₁ and Z₂,: the same or different from each other, represent F, Cl, Br or H.

Rₓ preferably is F or a perfluorinated alkyl group containing from 1 to 3 carbon atoms (e.g. CF₃ or C₂F₅) and most preferred is F.

The preferred fluorinated alkoximines of the present invention belong to the class of formula

CF₂=N-O-CFZ-CF₃ (I')

wherein Z represents F, Cl or H.

The following compounds belong to class (I'):

a)

CF₂=N-O-CF₂-CF₃ (II)

(pentafluoroethoxy)-carbonimide difluoride

b)

CF₂=N-O-CFCl-CF₃ (III)

(1-chloro-1,2,2,2-tetrafluoroethoxy)-carbonimide difluoride

c)

CF₂=N-O-CFH-CF₃ (IV)

(1,2,2,2-tetrafluoroethoxy)-carbonimide difluoride.

The starting materials for preparing the fluorinated alkoximines of formula (I) are the fluorinated alkoxy amines of general formula:

Rₓ-CF₂-NH-O-CZ₁Z₂-CF₃ (V)

wherein Rₓ, Z₁ and Z₂ are as defined above.

The fluorinated alkoxy amines (V), and a process for preparing them are disclosed in a copending patent application by the same applicant (EP-A 353 721).

According to said process, a fluorinated oxazetidine of formula 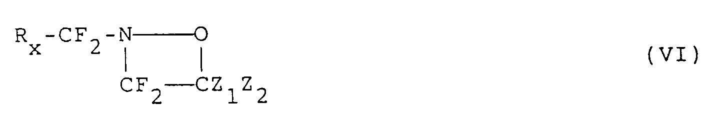
is reacted, at a temperature of from about -80°C to about +300°C, with HF, in the presence of a Lewis acid, preferably AsF₅.

According to the process fo the present invention, a fluorinated alkoxy amine of formula

Rₓ-CF₂-NH-O-CZ₁Z₂-CF₃ (V)

wherein Rₓ, Z₁ and Z₂ are as defined above, is reacted with KF at a temperature of from about 0°C to about 100°C.

A dehydrofluorination reaction takes place, whereby a fluorinated alkoximine of formula (I) is formed.
The temperature for said reaction preferably ranges from about 25°C to about 50°C.

The molar ratio of KF to the alkoxy amine of formula (V) usually is comprised within the range of from about 5 to about 50, preferably of from about 5 to about 15.

The reaction times usually are comprised within the range of from 0.5 hours to 30 hours, and, more frequently, of from 0.5 hours to 1 hour.

The fluorinated alkoximines of formula (I) can be converted into the corresponding N-chloro-, N-bromo- and N-fluoro-derivatives by a halogenation reaction, which will be described in the following.

The resulting compounds have the general formulae:

Rₓ-CF₂-NCl-O-CZ₁Z₂-CF₃ (VII)

Rₓ-CF₂-NBr-O-CZ₁Z₂-CF₃ (VIII)

Rₓ-CF₂-NF-O-CZ₁Z₂-CF₃ (IX)

Rₓ, Z₁ and Z₂ being defined as above.

The compounds of formulae (VII) and (VIII) are new, and are a further object of the present invention.
The preferred compounds of formulae (VII) and (VIII) are:

CF₃-NCl-O-CFZ-CF₃ (VII′)

CF₃-NBr-O-CFZ-CF₃ (VIII′)

wherein Z represents F, Cl or H.

Specific examples of said compounds are:

a′)

CF₃-NCl-O-CF₂CF₃ (X)

(N-chloro-N-(pentafluoroethoxy)-trifluoromethylamine)

b′)

CF₃-NCl-O-CFClCF₃ (XI)

N-chloro-N-(1-chloro-1,2,2,2-tetrafluoroethoxy)-trifluoromethylamine

c′)

CF₃-NCl-O-CFHCF₃ (XII)

N-chloro-N-(1,2,2,2-tetrafluoroethoxy)-trifluoromethylamine

a˝)

CF₃-NBr-O-CF₂CF₃ (XIII)

N-bromo-N-(pentafluoroethoxy)-trifluoromethylamine

b˝)

CF₃NBr-O-CFClCF₃ (XIV)

N-bromo-N-(1-chloro-1,2,2,2-tetrafluoroethoxy)-trifluoromethylamine

c˝)

CF₃-NBr-O-CFHCF₃ (XV)

N-bromo-N-(1,2,2,2-tetrafluoroethoxy)-trifluoromethylamine.

The fluorinated alkoximines of formula (I) are useful monomers in polymerization reactions. They are also useful intermediates for the preparation of their N-chloro-, N-bromo- and N-fluoro-derivatives.

The N-chloro-derivatives of formula (VII), and the N-bromo-derivatives of formula (VIII) are valuable telogens. The N-fluoro-derivatives of formula (IX) are useful catalysts for the polymerization of C₂F₄.

The fluorinated alkoximines of formula (I) can be chloro-fluorinated to yield the corresponding N-chloro-derivatives of formula (VII), the procedure being as follows: a fluorinated alkoximine of formula:

Rₓ-CF=N-O-CZ₁Z₂-CF₃ (I)

wherein Rₓ, Z₁ and Z₂ are defined as above,
is reacted with ClF at a temperature of from about -160°C to about +25°C. The reaction preferably is carried out in the presence of a catalyst, which catalyst is selected from fluorides of alkali metals or of alkaline earth metals or combinations thereof.

Preferred catalysts are CsF, RbF or KF; the most preferred catalyst is CsF.

The preferred reaction temperature is comprised within the range of from about -100°C to about +25°C.

The molar ratio of ClF to alkoximine of formula (I) usually ranges from about 1 to about 2 and, preferably is from about 1.0:1 to about 1.2:1.

The molar ratio of catalyst (e.g. CsF) to alkoximine of formula (I) generally is within the range of from about 0.5 to about 100, and preferably ranges from about 5 to about 10.

The fluorinated alkoximines of formula (I) can be bromofluorinated to yield their corresponding N-bromo-derivatives of formula (VIII), the procedure being as follows: a fluorinated alkoximine of the above formula (I) is reacted, at a temperature of from about -60°C to about +30°C, with Br₂ and a fluoride of an alkali metal or of an alkaline earth metal or combinations thereof.

The preferred reaction temperature is from about 0°C to about 30°C.

The metal fluoride preferably is CsF, RbF or KF and, most preferred, CsF.

The molar ratio of Br₂ to alkoximine of formula (I) usually ranges from about 1 to 100, and preferably is within the range of from about 3 to about 10.

The molar ratio of metal fluoride (e.g. CsF) to alkoximine of formula (I) usually ranges from about 2 to about 100, and preferably from about 10 to about 20.

The fluorinated alkoximines of formula (I) can be fluorinated to yield the corresponding N-fluoro-derivatives of formula (IX), the procedure being as follows: a fluorinated alkoximine of the above formula (I) is reacted with F₂ at a temperature of from about -196°C to about +25°C.

The reaction is preferably carried out in the presence of a catalyst selected from fluorides of alkali metals, alkaline earth metals or combinations thereof.

The preferred reaction temperature is from about -80°C to about +25°C.
The catalyst preferably is CsF, RbF or KF, most preferred CsF.

The molar ratio of F₂ to alkoximine of formula (I) generally is from about 1 to about 2, and preferably ranges from about 1.0 to about 1.2.

The molar ratio of the catalyst (e.g. CsF) to alkoximine of formula (I) commonly is comprised within the range of from about 0.5 to about 100, and preferably ranges from about 5 to about 10.

The above chlorofluorination, bromofluorination and fluorination reactions of the alkoximines of formula (I) may also be carried out in suitable solvents compatible with the reactants and with the reaction products.

The following examples are given in order to illustrate the present invention.

### EXAMPLE 1

This example describes the preparation of

CF₂=N-OCF₂-CF₃

by starting from CF₃-NH-OCF₂-CF₃.

Potassium fluoride (Aldrich) was dried and activated by melting it in a platinum crucible and subsequently grinding it in a ball-mill under a dry nitrogen atmosphere.
KF was stored and weighed inside the reactor in a "dry box", filled with nitrogen.
The reactor consisted of a 75 ml Hoke 304 bomb of stainless steel, provided with a Nupro SS-4JBR valve. Then, inside the reactor KF (1.15 g, 19.8 mmol) was weighed in the "dry box", together with three steel bearing balls.

Vacuum (5µm Hg) was applied, and 2.70 mmol of compound of formula

CF₃-N(H)-OCF₂-CF₃

were condensed in the reactor under static vacuum conditions (5 µm Hg), by means of a vacuum line of Pyrex glass, while the reactor was kept at a temperature of -196°C.

The temperature was increased to 24°C and the reactor was left standing, with occasional shaking, for 16 hours. The volatile contents of the reactor were then pumped into a "U"-shaped trap, cooled with liquid nitrogen, and were fractionated under dynamic vacuum (5 µm Hg) through traps kept at -50°C, -90°C, and -196°C. In the trap at -90°C, 2.49 mmol (yield 92.2%) of compound of formula

CF₂=N-O-CF₂CF₃

were collected.

This compound was characterized by IR, ¹⁹F-NMR and mass spectra.
For the IR characterization, a Perkin-Elmer 1430 apparatus equipped with a 7500 data station was used. The spectra were obtained in the gas phase with a 10-cm cell, provided with KCl windows attached with Halocarbon 1500 wax.

For the N.M.R. characterization, an IBM NR 200AF apparatus was used, operating at 200.13 MHz for ¹H and at 188.31 MHz for ¹⁹F.

The downfield chemical shifts with respect to internal (CH₃)₄Si and CFCl₃, respectively, are reported as positive.

The mass spectra were recorded on a Hewlett-Packard 5985 B apparatus, operating at 70 eV. CH₄ was used as CI gas. The temperatures were measured with a type J thermocouple.

The same characterization procedure was followed in Examples 2, 3, 4 and 5.
IR (4 torr): 2785(vw), 2742(vw), 1969(vw), 1850(vw), 1755(v_{C=N)},vs), 1394(sh,m), 1375(vs), 1237(vs), 1216(vs), 1195(s), 1108(vs), 1035(s), 926(w), 848(m), 812(vw), 744(m), 726(sh,w), 645(w), 527(w) cm⁻¹;
(m = medium; sh = shoulder; w = weak; s = strong; vs = very strong; vw = very weak)
¹⁹F-NMR F^{A}F^{X}C=N-OCF₂^{B}CF₃^{C} (CDCl₃, 24°C) δ A -54.4 (1F,br d), X -85.1 (1F, br d), B -93.9 (2F, d-q), C -84.8 ppm (3F, t), J_{AX} = 63.6, J_{BX} = 2.6, J_{BC} = 1.6, J_{AB} = J_{AC} =J_{CX} = 0 Hz.
Mass spectrum major peaks at m/z (EI): (M⁺), 180 (M-F⁺), 130 (CF₂=NOCF₂⁺), 119 (CF₂CF₃⁺), 69 (CF₃⁺), 64 (CF₂=N⁺), 50 (CF₂⁺), 47 (FCO⁺);
major peaks at m/z (CI): 200 (M+l⁺), 180 (M+l-HF⁺), 130 (CF₂=NOCF₂⁺), 119 (CF₃CF₂⁺).

### EXAMPLE 2

This example describes the preparation of

CF₂=N-OCFCl-CF₃

by starting from CF₃-NH-OCFCl-CF₃.

Potassium fluoride (Aldrich) as obtained, was melted, stored and weighed inside the reactor in a "dry box" filled with nitrogen.

The reactor was the same as in example 1. KF (2.25 g, 38.7 mmol) was weighed inside the reactor in the "dry box", with two steel bearing balls (diameter 3/8 inch) being added.

Then vacuum (5 µm Hg) was applied, and subsequently 3.87 mmol of compound of formula

CF₃-N(H)-OCFCl-CF₃

were added through a Pyrex vacuum line, under static vacuum conditions (5 µm Hg), while the reactor was kept at -196°C.

Thereupon, the temperature was increased to 24°C, and the reactor was then placed inside an oil bath and left standing, with stirring, at 60°C for 12 hours; during this time, the reactor was periodically withdrawn from the oil bath in order to shake it.

After allowing the reactor to cool down to 24°C, the volatile contents thereof were subsequently pumped into a "U"-shaped trap of Pyrex, cooled with liquid nitrogen, and were fractionated under dynamic vacuum (5 µm Hg), through a series of traps of -70°C, -120°C, and 196°C. In the trap at -120°C, a compound of formula

CF₂=N-OCFCl-CF₃

was collected (2.12 mmol; yield 54.8%).

This compound was characterized as follows:
IR (4 torr): 2778(vw), 2742(vw), 1751(v_{C=N},vs), 1375(vs), 1331(s), 1286(sh,vw), 1231(vs), 1155(vs), 1098(vs), 1032(s), 998(vs), 946(vw), 912(m), 863(vw), 828(w), 768(m), 727(m), 700(sh,w), 642(m), 606(vw), 530(m) cm⁻¹;
¹⁹F-NMR F^{A}F^{X}C=N-OCF₂^{B}ClCF₃^{C} (CDCl₃, 24°C) δ A -53.9 (1F,d-d), X -84.5 (1F, br d), B -79.9 (1F, q-t), C -83.2 ppm (3F, d), J_{AX} = 63.8, J_{AB} = J_{BX} = 1.8, J_{BC} = 3.0, J_{AC} = J_{CX} = 0 Hz.
Mass spectrum major peaks at m/z (EI): 215/217 (M⁺), 196/198 (M-F⁺), 180 (M-Cl⁺), 146/148 (M-CF₃⁺), 135/137 (CF₃CFCl⁺), 130 (M-CF₂Cl⁺), 85/87 (CF₂Cl⁺), 69 (CF₃⁺), 64 (CF₂=N⁺), 50 (CF₂⁺), 47 (COF⁺);
major peaks at m/z (CI): 216/218 (M+l⁺), 196/198 (M+l-HF⁺), 180 (M+l-HCl⁺), 146/148 (M-CF₃⁺), 135/137 (CF₃CFCl⁺), 130 (M-CF₂Cl⁺).

### EXAMPLE 3

This example describes the preparation of:

CF₂=N-OCHF-CF₃

by starting from CF₃-NH-OCHF-CF₃.

The preparation of CF₂=N-OCHF-CF₃ was carried out by following the procedure previously adopted for the preparation of CF₂=N-OCFCl-CF₃, with the following modifications:
- CF₃-N(H)-OCHF-CF₃:: 2.71 mmol
- KF:: 1.10 g (19.0 mmol)
- Reaction time at 60°C:: 17 hours
- Fractionation:: traps at -80°C, -120°C and -196°C.
CF₂=N-OCHF-CF₃ (2.00 mmol) was collected in the trap at -120°C, with a yield of 73.8%.
CF₂=N-OCHF-CF₃ was characterized as follows:
IR (3 torr): 2998 (v_{C-H},m), 1750(v_{C-N},vs), 1705(sh,vw), 1441(vw), 1412(m), 1369(vs), 1329 (vw), 1290(s), 1250(w), 1214(vs), 1192(vs), 1143(vs), 1091(s), 1027(s), 961(m), 928 (vw), 903(vw), 872(m), 729(m), 677(m), 637(w), 602(sh,vw), 584(sh,vw), 566(w), 534(w) cm⁻¹.
¹⁹F-NMR F^{A}F^{X}C=N-OCHF^{B}CF₃^{D} (CDCl₃, 24°C): ¹H δ 5,8 (d-q-t), ¹⁹F δ A -56.1 (1F,d-d), X -87.7 (1F, br d), B -143.8 (1F, d-q-d-d), D -82.4 ppm (3F, d-d), J_{HA}=J_{HX} = 0.6, J_{HB} = 57.6, J_{HC} = 3.2, J_{AX} = 57.6, J_{AB} = 3.0, J_{BX} = 0.6, J_{BC} = 6.1, J_{AC}=J_{CX} = 0 Hz.
Mass spectrum: major peaks at m/z (EI): 181(M⁺), 162 (M-F⁺), 112 (M-CF₃⁺), 101 (CF₃CHF⁺), 69 (CF₃⁺), 64 (CF₂=N⁺), 50 (CF₂⁺);
major peaks at m/z (CI): 182 (M+l⁺), 162 (M+l-HF⁺), 112 (M-CF₃⁺).

### EXAMPLE 4

This example describes the preparation of

CF₃-NCl-OCF₂CF₃

by starting from CF₂=N-OCF₂CF₃ and ClF.

The reactor was that employed in example 1.
Vacuum was applied (5 µm Hg) and the metal vacuum line as well as the reactor were treated with four successive portions of ClF₃ gas (100 torr). The ClF₃ was added to the system under vacuum, and then left standing at 22°C, each time for about 10 minutes.

The reactor was evacuated and cooled to -196°C, and 0.61 mmol of compound of formula

CF₂=NOCF₂CF₃

was condensed therein under static vacuum conditions (5 µm Hg). Thereafter, 0.79 mmol of ClF were added.

The temperature of the reactor was then allowed to slowly increase inside a Dewar bottle which initially was at -196°C. 9.5 hours later, the temperature was 15°C, and the reactor was removed from the Dewar bottle and was left standing at 22°C for half an hour.

The reactor was then cooled down to -120°C, and any material volatile at this temperature was removed under dynamic vacuum (5 µm Hg). Thereafter the residue was fractionated by pumping it through a series of cold traps kept at temperatures of -55°C, -100°C, and -196°C, respectively. 0.57 mmol (yield: 94%) of compound of formula

CF₃-N(Cl)OCF₂-CF₃

were collected in the trap at -100°C.

CF₃-N(Cl)OCF₂-CF₃ was characterized as follows:
IR (2 torr): 2047(vw), 1875(vw), 1392(m), 1276(vs), 1243(vs), 1218(s), 1187(vs), 1097(vs), 953(w), 879(m), 853(w), 798(vw), 766(w), 741(m), 693(vw), 672 (m), 523(w) cm⁻¹.
¹⁹F-NMR CF₃^{C}N(Cl)-OCF^{A}F^{B}CF₃^{CD} (CDCl₃, 24°C) δ A -94.3, B -97.6 (2F, rather broad typical AB pattern), C -76.5 (3F, t), D -84.8 ppm (3F, t), J_{AB} = 144, J_{AC} = J_{BC} = 2.7, J_{AD} = J_{BD} = 1.7, J_{CD} = 0 Hz.
Mass spectrum major peaks at m/z (EI): 219 (M-Cl⁺), 131 (CF₂=NOCF₂⁺), 119 (CF₂CF₃⁺), 100 (CF₃CF⁺), 99 (CF₃NO⁺), 83 (CF₃N⁺), 69 (CF₃⁺), 64 (CF₂=N⁺), 50 (CF₂⁺); major peaks at m/z (CI): 254/256 (M+l⁺), 234/236 (M+l-HF⁺), 219 (M+l-Cl⁺), 218 (M+l-HCl⁺), 180 (FCN-OC₂F₅⁺), 135 (CF₃CF₂O⁺), 130 (CF₂=NOCF₂⁺), 119 (CF₃CF₂).

### EXAMPLE 5

This example describes the preparation of

CF₃-NBr-OCF₂CF₃

by starting from CF₂=N-OCF₂CF₃, Br₂ and CsF.

Cesium fluoride (Cabot) was dried and activated by melting it inside a platinum crucible, followed by grinding inside a ball mill under a dry nitrogen atmosphere. CsF was handled and weighed inside the reactor in a nitrogen-filled "dry box". Bromine (Fisher) was stored over P₄O₁₀ in a bulb provided with a glass/Teflon® stopcock.
The reactor was a 50 ml bulb accommodated inside a screw-top fitting ("Ace-Thred", Ace Glass Co.), which was tightly sealed by means of an O-ring compressed by a Teflon® insulating gasket through which a tube provided with a glass/Teflon® stopcock protruded.

CsF (0.76 g, 5.0 mmol) was weighed inside the reactor in the "dry box", and a Teflon®-coated magnetic stirring bar was added.

The bulb was then subjected to vacuum (5 µm Hg, with the same vacuum being applied in all of the subsequent operating steps to be conducted under vacuum), and 7.00 mmol of bromine were condensed therein by static vacuum, through a Pyrex vacuum line, the reactor being maintained at a temperature of -196°C. The CsF/Br₂ mixture was stirred at 22°C for 4 hours.

Then the reactor was cooled again with liquid nitrogen, and CF₂=N-OCF₂CF₃ (1.00 mmol) was added through the glass line, under static vacuum, by operating in the dark. The reactor was heated to 22°C, was wrapped with an aluminum foil and was left standing for 16 hours with stirring.

Volatile materials were then pumped into a "U"-shaped Pyrex trap cooled with liquid nitrogen. Ethylene (Matheson C.P., 3.00 mmol) was allowed to condense inside the trap, under static vacuum. The liquid nitrogen bath was then removed from the trap (volume = 73 ml), and the contents of the trap were allowed to warm to 22°C, in order to cause the excess Br₂ to react. The resulting mixture, consisting of CF₃-N(Br)-OCF₂CF₃, BrCH₂CH₂Br and unreacted CH₂=CH₂, was then fractionated, under dynamic vacuum conditions, through a series of traps at -55°C, -135°C and -196°C. The desired product, CF₃-N(Br)-OCF₂CF₃ (0.99 mmol, yield 99%), condensed in the trap at -135°C.

CF₃-N(Br)-OCF₂CF₃ was characterized as follows:
IR (2 torr, cell with AgCl plates): 1390(m), 1269(vs), 1241(vs), 1214(s), 1184(vs), 1097(vs), 948(m), 876(m), 848(w), 754(m), 723(m), 664(m), 634(vw), 520(vw) cm⁻¹.
¹⁹F-NMR CF₃^{C}N(Br)-OCF^{A}F^{B}CF₃^{D} (CCl₄, (CD₃)₂SO, coaxial, 24°C): δ A -95.4, B -98.9 (2F, very broad typical AB pattern, A and B are the highest values of very broad peaks), C -75.1 (3F, t), D -85.2 ppm (3F, t), J_{AB} = not determined, J_{AC}=J_{BC} = 3.0, J_{AD}=J_{BD} = 1.6, J_{CD} = 0. In order to determine the values of δ_{A}, δ_{B} and J_{AB}, a low-temperature spectrum was recorded in CDCl₃ at -30°C. The variable temperature unit was a Bruker B-VT 1000 with a "factor-specified" precision of ±0.5 K.

Under these conditions, the N.M.R. spectrum was as follows: δA -94.8 (d-q-q), B -99.3 (d), C -74.7 (d), D -84.9 ppm (t), J_{AB} = 141.8, J_{AC} = 5.1, J_{AB}=J_{BD} = 1.4, J_{BC}=J_{CD} = 0 Hz.
Mass spectrum major peaks at m/z (EI): 297/299 (M⁺), 218 (M-Br⁺), 178/180 (CF₃=NBrO⁺), 164 (CF₃NBr⁺), 130 (CF₃NOCF⁺), 119 (CF₂CF₃⁺), 79/81 (Br⁺), 69 (CF₃⁺), 64 (CF₂=N⁺), 50 (CF₂⁺); 47 (COF⁺);
major peaks at m/z (CI): 298/300 (M+l⁺), 278/280 (M+l-HF⁺), 218 (M+l-HBr⁺), 164 (CF₃NBr⁺), 199 (CF₃CF₂⁺).

### EXAMPLE 6

This example describes the preparation of

CF₃-NF-OCF₂CF₃

by starting from CF₂=N-OCF₂CF₃ and F₂.

The reaction vessel was a 150 ml Hoke bomb of stainless steel, equipped with a Nupro SS-4JBR valve. A vacuum of 5 µm Hg was applied (said vacuum being also applied in all subsequent steps to be carried out under vacuum) and then 1 atm of F₂ was introduced through a manifold of stainless steel, in order to passivate the equipment.
After 3 hours at 22°C, the F₂ was evacuated.
Then, before use, commercial F₂ (Air Products) was caused to flow through a NaF scrubber.

The passivated reactor was cooled to -196°C, and CF₂=N-OCF₂CF₃ (1.00 mmol) was condensed therein by means of a static vacuum, through a vacuum line of Pyrex glass. The reactor was then connected to a stainless steel line, and F₂ (1.05 mmol, 5% excess) was added, still at the temperature of liquid nitrogen.

Thereupon, the reactor was placed inside a cold empty Dewar bottle, from which liquid nitrogen had been removed shortly before. The upper end of the Dewar bottle was covered with an aluminum foil. The temperature of the reactor increased from -196°C to 22°C within about 17 hours.

The reactor was then removed from the Dewar bottle, and was left standing at 23°C for 6 hours, whereupon the reactor was cooled again with liquid nitrogen, and a trace of volatile matter was pumped off at -196°C.

Then, with the reactor temperature being maintained at 23°C, volatile materials were pumped into a "U"-shaped Pyrex trap cooled with liquid nitrogen. The fractionation, under dynamic vacuum, through a series of traps cooled at -100°C, -135°C and -196°C, afforded a compound of formula

CF₃-N(F)-OCF₂CF₃

(0.84 mmol, yield 84%) inside the trap at -135°C.

## Claims

1. Fluorinated alkoximines of general formula:
Rₓ-CF=N-O-CZ₁Z₂-CF₃ (I)
wherein:
Rₓ is F or a perhaloalkyl group containing from 1 to 3 carbon atoms, and
Z₁ and Z₂, the same or different from each other, represent F, Cl, Br or H.

2. Compounds according to claim 1, wherein Rₓ is F or a perfluoroalkyl group containing from 1 to 3 carbon atoms and preferably is F.

3. Compounds according to claim 1, having the general formula:
CF₂=N-O-CFZ-CF₃ (I′)
wherein Z represents F, Cl or H.

4. Fluorinated N-chloro- or -bromo-alkoxy-amines of general formulae:
Rₓ-CF₂-NCl-O-CZ₁Z₂-CF₃ (VII)
and
Rₓ-CF₂-NBr-O-CZ₁Z₂-CF₃ (VIII)
wherein:
Rₓ is F or a perhaloalkyl group containing from 1 to 3 carbon atoms, and
Z₁ and Z₂, the same or different from each other, represent F, Cl, Br, H or a perfluoroalkyl group containing from 1 to 3 carbon atoms.

5. Compounds according to claim 4, wherein Rₓ is F or a perfluoroalkyl group containing from 1 to 3 carbon atoms and preferably is F.

6. Compounds according to claim 4, having the formula:
CF₃-NY-O-CFZ-CF₃
wherein Z is F, Cl or H and Y represents Cl or Br.

7. Process for preparing the compounds of formula (I) according to claim 1, characterized in that a fluorinated alkoxy-amine of general formula
Rₓ-CF₂-NH-O-CZ₁Z₂-CF₃ (V)
wherein Rₓ, Z₁ and Z₂ are defined as in any one of claims 1 and 2,
is reacted with KF at a temperature of from 0°C to 100°C.

8. Process for preparing N-chloro-substituted alkoxy-amines of formula (VII) according to claim 4, characterized in that a fluorinated alkoximine of general formula:
Rₓ-CF=N-O-CZ₁Z₂-CF₃ (I)
wherein Rₓ, Z₁ and Z₂ are defined as in any one of claims 1 and 2,
is reacted with ClF at a temperature of from about -160°C to about +25°C.

9. Process according to claim 8, characterized in that the reaction is carried out in the presence of a catalyst selected from fluorides of alkali or alkaline earth metals.

10. Process for preparing N-bromo-substituted alkoxy-amines of formula (VIII) according to claim 4, characterized in that a fluorinated alkoximine of general formula:
Rₓ-CF=N-O-CZ₁Z₂-CF₃ (I)
wherein Rₓ, Z₁ and Z₂ are defined as in any one of claims 1 and 2,
is reacted, at a temperature of from about -60°C to about +30°C, with Br₂ and a fluoride of an alkali or alkaline earth metal.

11. Process for preparing N-fluoro-substituted alkoxy-amines of general formula
Rₓ-CF₂-NF-O-CZ₁Z₂-CF₃ (IX)
wherein Rₓ, Z₁ and Z₂ are defined as in any one of claims 1 and 2, characterized in that a fluorinated alkoximine of general formula:
Rₓ-CF=N-O-CZ₁Z₂-CF₃ (I)
wherein Rₓ, Z₁ and Z₂ are as defined above, is reacted with F₂ at a temperature of from about -196°C to about +325°C.

12. Process according to claim 11, characterized in that the reaction is carried out in the presence of a catalyst selected from fluorides of alkali or alkaline earth metals.

## Patentansprüche

1. Fluorierte Alkoximine der allgemeinen Formel:
Rₓ-CF=N-O-CZ₁Z₂-CF₃ (I)
worin:
Rₓ für F oder eine Perhalogenalkylgruppe, die 1 bis 3 Kohlenstoffatome enthält, steht, und
Z₁ und Z₂, gleich oder verschieden voneinander, F, Cl, Br oder H darstellen.

2. Verbindungen nach Anspruch 1, worin Rₓ für F oder eine Perfluoralkylgruppe mit 1 bis 3 Kohlenstoffatomen steht und vorzugsweise F darstellt.

3. Verbindungen nach Anspruch 1 mit der allgemeinen Formel:
CF₂=N-O-CFZ-CF₃ (I')
worin Z für F, Cl oder H steht.

4. Fluorierte N-Chlor- oder -Brom-Alkoxyamine der allgemeinen Formeln:
Rₓ-CF₂-NCl-O-CZ₁Z₂-CF₃ (VII)
und
Rₓ-CF₂-NBr-O-CZ₁Z₂-CF₃ (VIII)
worin:
Rₓ für F oder eine Perhalogenalkylgruppe, die 1 bis 3 Kohlenstoffatome enthält, steht und
Z₁ und Z₂, gleich oder verschieden voneinander, F, Cl, Br, H oder eine Perfluoralkylgruppe, die 1 bis 3 Kohlenstoffatome enthält, darstellen.

5. Verbindungen nach Anspruch 4, worin Rₓ für F oder eine Perfluoralkylgruppe, die 1 bis 3 Kohlenstoffatome enthält, steht und vorzugsweise F ist.

6. Verbindungen nach Anspruch 4 mit der Formel:
CF₃-NY-O-CFZ-CF₃
worin:
Z für F, Cl oder H steht und
Y für Cl oder Br steht.

7. Verfahren zur Herstellung der Verbindungen der Formel (I) gemäß Anspruch 1, dadurch gekennzeichnet, daß ein fluoriertes Alkoxyamin der allgemeinen Formel
Rₓ-CF₂-NH-O-CZ₁Z₂-CF₃ (V)
worin Rₓ, Z₁ und Z₂ wie in irgendeinem der Ansprüche 1 und 2 definiert sind,
mit KF bei einer Temperatur von 0°C bis 100°C umgesetzt wird.

8. Verfahren zur Herstellung von N-Chlor-substituierten Alkoxyaminen der Formel (VII) gemäß Anspruch 4, dadurch gekennzeichnet, daß ein fluoriertes Alkoximin der allgemeinen Formel:
Rₓ-CF=N-O-CZ₁Z₂-CF₃ (I)
worin Rₓ, Z₁ und Z₂ wie in irgendeinem der Ansprüche 1 und 2 definiert sind,
mit ClF bei einer Temperatur von etwa -160°C bis etwa +25°C umgesetzt wird.

9. Verfahren nach Anspruch 8, dadurch gekennzeichnet, daß die Umsetzung in Anwesenheit eines aus Fluoriden von Alkali- oder Erdalkalimetallen ausgewählten Katalysators durchgeführt wird.

10. Verfahren zur Herstellung von N-Brom-substituierten Alkoxyaminen der Formel (VIII) gemäß Anspruch 4, dadurch gekennzeichnet, daß ein fluoriertes Alkoximin der allgemeinen Formel:
Rₓ-CF=N-O-CZ₁Z₂-CF₃ (I)
worin Rₓ, Z₁ und Z₂ wie in irgendeinem der Ansprüche 1 und 2 definiert sind,
bei einer Temperatur von etwa -60°C bis etwa +30°C mit Br₂ und einem Fluorid eines Alkali- oder Erdalkalimetalls umgesetzt wird.

11. Verfahren zur Herstellung von N-Fluor-substituierten Alkoxyaminen der allgemeinen Formel
Rₓ-CR₂-NF-O-CZ₁Z₂-CF₃ (IX)
worin Rₓ, Z₁ und Z₂ wie in irgendeinem der Ansprüche 1 und 2 definiert sind, dadurch gekennzeichnet, daß ein fluoriertes Alkoximin der allgemeinen Formel:
Rₓ-CF=N-O-CZ₁Z₂-CF₃ (I)
worin Rₓ, Z₁ und Z₂ wie oben definiert sind, bei einer Temperatur von etwa -196°C bis etwa +325°C mit F₂ umgesetzt wird.

12. Verfahren nach Anspruch 11, dadurch gekennzeichnet, daß die Reaktion in Anwesenheit eines aus Fluoriden von Alkali- oder Erdalkalimetallen ausgewählten Katalysators durchgeführt wird.

## Revendications

1. Alcoximines fluorées de formule générale:
Rₓ-CF=N-O-CZ₁Z₂-CF₃ (I)
dans laquelle:
Rₓ représente F ou un groupe perhaloalkyle contenant de 1 à 3 atomes de carbone, et
Z₁ et Z₂, identiques ou différents l'un de l'autre, représentent F, CI, Br ou H.

2. Composés selon la revendication 1, caractérisés en ce que Rₓ représente F ou un groupe perfluoroalkyle contenant de 1 à 3 atomes de carbone et représente de préférence F.

3. Composés selon la revendication 1, répondant à la formule générale:
CF₂=N-O-CFZ-CF₃ (I')
dans laquelle:
Z représente F, Cl ou H.

4. N-chloro-, ou N-bromo-alcoxyamines fluorées de formule générale:
Rₓ-CF₂-NCl-O-CZ₁Z₂-CF₃ (VII)
et
Rₓ-CF₂-NBr-O-CZ₁Z₂-CF₃ (VIII)
dans laquelle:
Rₓ représente F ou un groupe perhaloalkyle contenant de 1 à 3 atomes de carbone, et
Z₁ et Z₂, identiques ou différents l'un de l'autre, représentent F, Cl, Br, H ou un groupe perfluoroalkyle renfermant de 1 à 3 atomes de carbone.

5. Composés selon la revendication 4, caractérisés en ce que Rₓ représente F ou un groupe perfluoroalkyle renfermant de 1 à 3 atomes de carbone et représente de préférence F.

6. Composés selon la revendication 4, répondant à la formule générale:
CF₃-NY-O-CFZ-CF₃
dans laquelle:
Z représente F, Cl ou H, et
Y représente Cl ou Br.

7. Procédé de préparation des composés de formule (I) selon la revendication 1, caractérisé en ce que l'on fait réagir une alcoxyamine fluorée de formule générale:
Rₓ-CF₂-NH-O-CZ₁Z₂-CF₃ (V)
dans laquelle:
Rₓ, Z₁ et Z₂, sont tels que définis dans l'une quelconque des revendications 1 et 2,
avec KF à une température entre 0°C et 100°C.

8. Procédé de préparation d'alcoxyamines de N-chloro substituées de formule (VII) selon la revendication 4, caractérisé en ce que l'on fait réagir une alcoximine fluorée de formule générale:
Rₓ-CF=N-O-CZ₁Z₂-CF₃ (I)
dans laquelle:
Rₓ, Z₁ et Z₂, sont tels que définis dans l'une quelconque des revendications 1 et 2,
avec ClF à une température d'environ -160°C à environ °25°C.

9. Procédé selon la revendication 8, caractérisé en ce que la réaction est mise en oeuvre en présence d'un catalyseur choisi parmi des fluorures de métaux alcalins ou alcalino-terreux.

10. Procédé de préparation d'alcoximines N-bromo substituées de formule (VIII) selon la revendication 4, caractérisé en ce que l'on fait réagir une alcoximine fluorée de formule générale:
Rₓ-CF=N-O-CZ₁Z₂-CF₃ (I)
dans laquelle:
Rₓ, Z₁ et Z₂, sont tels que définis dans l'une quelconque des revendications 1 et 2,
à une température d'environ -60°C à environ 30°C avec Br₂ et un fluorure d'un métal alcalin ou alcalino-terreux.

11. Procédé de préparation d'alcoximines N-fluoro substituées de formule générale:
Rₓ-CF₂-NF-O-CZ₁Z₂-CF₃ (IX)
dans laquelle:
Rₓ, Z₁ et Z₂, sont tels que définis dans l'une quelconque des revendications 1 et 2,
caractérisé en ce que l'on fait réagir une alcoximine de formule générale:
Rₓ-CF=N-O-CZ₁Z₂-CF₃ (I)
dans laquelle:
Rₓ, Z₁ et Z₂, sont tels que définis ci-dessus,
avec F₂ à une température d'environ -196°C à environ °325°C.

12. Procédé selon la revendication 11, caractérisé en ce que la réaction est mise en oeuvre en présence d'un catalyseur choisi parmi des fluorures de métaux alcalins ou alcalino-terreux.
